# EUROPEAN PATENT SPECIFICATION

(11) **EP 0 589 818 B1**
(45) Date of publication and mention of the grant of the patent: **09.09.1998**
(21) Application number: 93500100.8
(22) Date of filing: 09.07.1993
(51) Int. Cl.: C02F 3/02, C02F 3/34, B09B 3/00, C02F 3/12, C12R 1/385, C12P 1/04, C12R 1/38

(54) **Microorganisms and process for the biological removal of nitrated derivatives**
Mikroorganismen und Verfahren zur biologischen Entfernung von Nitratderivaten
Microorganismes et procédé d'élimination biologique de dérivés de nitrate

(30) Priority: 10.07.1992 ES 9201436
(43) Date of publication of application: 30.03.1994
(73) Proprietor: UNION ESPANOLA DE EXPLOSIVOS S.A., E-28006 Madrid (ES)
(72) Inventor: Ramos Martin, Juan Luis, E-18191 Pindos Genil (Granada) (ES); Duque Martin de Oliva, Estrella, E-18191 Pindos Genil (Granada) (ES)
(74) Representative: Garcia Cabrerizo, Francisco

(56) References cited:
- WO-A-91/15440
- WO-A-92/19373
- US-A- 5 085 998
- DATABASE WPI Section Ch, Week 8912, Derwent Publications Ltd., London, GB; Class D04, AN 89-091696 & SU-A-1 423 585 (MAKEEVKA ENG. CONS.)

## Description

### Scope of the invention

The present invention generally relates to a process for the biological removal of nitrated derivatives. In particular, the invention provides a process for the removal of nitroaromatic compounds, such as 2,4-, 2,6-dinitrotoluene and 2,4,6-trinitrotoluene, by employing bacteria that are capable of utilizing said compounds as sources of carbon and/or nitrogen, thereby avoiding the environmental pollution caused by said nitroaromatic compounds.

### Background to the invention

Industry in general, and the explosives industry in particular, manufactures large quantities of various compounds substituted with nitro groups which are employed as explosives and propellants. The wash effluents of explosives production plants contain various nitro-organic compounds which, if not adequately treated, constitute a source of environmental pollution and which therefore need to be degraded since many of these compounds are also toxic in moderate concentrations and mutagenic and, therefore, suspected cancer-inducing agents.

In the effluents of explosives plants, especially in those that manufacture 2,4,6-trinitrotoluene (TNT), small quantities of nitrated compounds such as nitroglycerine, diethyleneglycol dinitrate and diethyleneglycol mononitrate can be detected, as well as variable quantities of nitroaromatic compounds, such as 2,4,6-trinitrotoluene (TNT), 2,4-dinitrotoluene (2,4-DNT), 2,6-dinitrotoluene (2,6-DNT), together with other mononitrotoluenes.

The present methods of treating effluents from explosives production plants include (i) infinite dilution of same with water and/or (ii) incineration. The first alternative entails the problem of the constant addition of pollutants to rivers, underground water and soil, which makes it unsatisfactory, whereas the second alternative entails considerable cost as well as potential risks due to the acidity of the samples, which makes it undesirable.

Microbiology in general and biotechnology in particular can offer a biological alternative to the removal of these nitroaromatic compounds through their utilization by suitable microorganisms as a source of C and/or N. Various studies have dealt with the possibility of treating biologically effluents that contain nitroaromatic compounds, including TNT and various compounds of DNT. The following studies in particular should be mentioned:

Carpenter et al. (Applied and Environmental Microbiology 35: 949-954, 1978) and Traxler (Dev. Ind. Microbiol. 16: 71-76) used nondefined microorganisms, which gives rise to problems of reproducibility of treatment. Moreover, they reported in a few cases the accumulation of nitrite, which is an undesired product;

Fernando et al. (Applied and Environmental Microbiology 56: 1666-1671, 1990) and Parrish et al. (Applied and Environmental Microbiology 34: 232-233, 1977) have demonstrated that various fungi can utilize TNT as a source of C and N or biotransform TNT into other reduced forms. The treatment of effluents with fungi, however, has a series of disadvantages related to their mycelia, which may cause suspension problems, ventilation system blockage and nonspecific pollution.

Spanggord et al. (Applied and Environmental Microbiology 57:3200-3205, 1991) have isolated bacteria of the genus Pseudomonas that can utilize 2,4-DNT as a source of C, but these bacteria accumulate nitrite and do not utilize either 2,6-DNT or TNT as a source of C.

It would therefore be advantageous to have available microorganisms that are capable of utilizing the nitroaromatic compounds (mainly 2,4-DNT, 2,6-DNT and TNT) in the effluents of explosives manufacturing plants as a source of C and/or N in order to remove the biologically, thereby overcoming the aforementioned disadvantages and avoiding the environmental problems associated with the uncontrolled disposal of these effluents or with their inadequate treatment.

Consequently, an object of the present invention is the isolation and characterization of microorganisms capable of degrading 2,4-DNT, 2,6-DNT and TNT. Said microorganisms constitute an additional object of this invention.

A second object of this invention comprises a process for the biological removal of nitroaromatic compounds, such as 2,4-DNT, 2,6-DNT and TNT, contained in industrial effluents and/or in polluted soil, by employing the microorganisms provided by this invention. Additionally, said microorganisms are also suitable for destroying excess TNT from arsenals or explosives plants.

### Detailed description of the invention

The invention provides a process for the biological removal of nitroaromatic compounds such as 2,4-DNT, 2,6-DNT and TNT, by employing microorganisms capable of degrading said nitroaromatic compounds.

By way of summary, the invention comprises on the one hand the isolation and characterization of microorganisms capable of degrading the aforesaid nitroaromatic compounds and, on the other hand, the employment of the isolated microorganisms to remove the 2,4-DNT, 2,6-DNT and TNT present not only in industrial plant effluents but also in soil polluted with said compounds. Additionally, said microorganisms may also be employed to destroy excess TNT.

### Isolation and characterization of microorganisms capable of degrading 2,4-DNT, 2,6-DNT and TNT

Isolation of microorganisms capable of utilizing 2,4-DNT, 2,6-DNT and TNT as sources of C and/or N was carried out in the following manner. A sample of soil situated near a plant generating effluents loaded with said nitroaromatic compounds was suspended in an M9-type minimum culture medium or similar without N source (Abril et al., Journal Bacteriology 57: 6782-6790, 1989). To said suspension was added the nitroaromatic compound in question, either alone (in the case it might be required to utilize same as a source of C and N) or combined with an appropriate quantity of a source of C (in the case where the nitroaromatic compound might be required to be utilized exclusively as a source of N). In the latter case, a sugar, preferably fructose or glucose or an appropriate organic acid, preferably acetic or succinic acid, may be utilized as a source of C. The resulting suspension was then incubated at temperatures of between 15° and 42°C, preferably between 25° and 30°C, with optional agitation, and, after a week of incubation, suitable dilutions were applied to plates of a selective solid medium such as that constituted by M9 without source of N, noble agar, nitroaromatic compound and, if required, an additional source of C. After an incubation period of between 24 hours and one week, the isolated colonies were purified and their phenotype established by cultivating them in the aforementioned media. In Example 1, the isolation of bacteria capable of utilizing 2,4-DNT, 2,6-DNT and TNT as sources of C and/or N is described.

Following a microbiological type analysis, the isolated bacteria could be classified as belonging to the genus Pseudomonas, and probably belonging to the species Pseudomonas aeruginosa. The isolated bacteria exhibited a bacillary shape, possessed a polar flagellum and exhibited yellow pigmentation when cultivated on LB medium plates (Maniatis et al. Molecular Cloning, A Laboratory Manual, Cold Spring Harbor Laboratory, NY, 1982). In an M9-type minimum medium, fructose, glucose, acetic acid and succinic acid may be utilized as a source of C, and ammonia, nitrate and nitrite as a source of N. A culture of these bacteria called Pseudomonas aeruginosa ClonA was deposited in the National Collection of Type Cultures (NCTC) in the United Kingdom on 27 April 1992 under Accession No. NCTC 12690.

Once isolated and purified, the bacteria colonies capable of utilizing 2,4-DNT, 2,6-DNT and TNT as sources of C and/or N may be utilized to remove nitroaromatic compounds present not only in industrial plant effluents but also in soil polluted with said compounds. These bacteria may likewise be used to destroy excess TNT.

### Process for the biological removal of 2,4-DNT, 2,6-DNT and TNT

A primary application of the bacteria provided by this invention is their employment in a process for removing biologically 2,4-DNT, 2,6-DNT and TNT contained in an industrial effluent. Said process basically comprises the following stages:
a) neutralizing the effluent to be treated;
b) supplementing with the necessary nutrients for optimal functioning of the process;
c) inoculating the effluent to be treated with cultures of microorganisms that can degrade the nitroaromatic compounds until they disappear;
d) withdrawing microorganisms which, if desired, may be reutilized in a further effluent treatment cycle; and
e) disposing of the purified water.

Additionally, if the purified water contains inorganic N, it may be biologically treated a second time to remove said N by employing suitable microorganisms before finally disposing of the purified water.

As mentioned previously, the process starts with the effluent neutralizing stage. Effluents that are purifiable by this process may be effluents from any industrial process in which the aforementioned nitroaromatic compounds are generated, such as effluents from explosives manufacturing plants. Water that is polluted with said nitroaromatic compounds may likewise be treated by this process.

Waste water from explosives production plants is generally acid water which can be neutralized by adding any alkali, preferably a hydroxide or an alkaline or alkaline earth carbonate or bicarbonate, so that said water achieves a pH of between 4.5 and 10.5 after it is neutralized.

Subsequently, and after analysis of the neutralized effluent, there is added necessary nutrients such as phosphate and any other nutrient or micronutrient that is required for the optimum functioning of the process. As an example, there may be added suitable quantities of a solution of A9 micronutrients (Abril et al. cited above) together with suitable quantities of Fe, Mg, Co and Mo (generally of a micromolar order of concentration). In either case, the choice and quantity of nutrients and micronutrients to be added will depend on the composition of the effluent to be treated and on the microbiological demand.

The neutralized and duly supplemented effluent is then inoculated with a culture of bacteria provided by this invention (Pseudomonas aeruginosa ClonA) that are capable of degrading the nitroaromatic compounds 2,4-DNT, 2,6-DNT and TNT. The bacterial load may range from between 0.01 and 0.1 units of absorbency at 660 nm per ml of culture, and said charge should preferably be around 0.03 absorbency units at 660 nm per ml of culture.

Incubation should be carried out at a temperature of between 20°C and 37°C, with an optional aeration of up to 2 litres of air per litre of culture. During the course of incubation the content in nitroaromatic compounds present in the effluent should be measured periodically using chromatographic techniques such as gas chromatography or HPLC, and this stage is considered finished once said nitroaromatic compounds can no longer be detected.

The bacteria are subsequently removed by floculation or by any other method which, if desired, permits them to be reutilized in a further process, and the purified water is removed and can be freely disposed of.

As previously mentioned, the bacteria provided by this invention can utilize the aforementioned nitroaromatic compounds as a source of C and/or N. When these bacteria utilize them as a source of C, the compounds are incorporated into their metabolic cycle and they are finally oxidized, to obtain CO₂ and H₂O. When they utilize them as a source of N, toluene and its derivatives are formed which can be recovered or disposed of or alternatively treated with bacteria that utilize toluene as a source of C.

This process may be carried out as a continuous or batch process, as well as in large-scale pools or fermenters. When it is carried out continously, the flow and composition of the ingoing and outgoing effluent must be adequately regulated, as must the concentration of bacterial culture and the addition of necessary nutrients and micronutrients.

A second application of the bacteria provided by this invention is their employment in a process for removing biologically the aforesaid nitroaromatic compounds that are present in topsoil polluted with said compounds. The said process comprises the following stages:
a) ventilation of the soil by deep ploughing;
b) initial injection of microorganisms that can degrade said nitroaromatic compounds in a liquid medium;
   and
c) successive injections of the aforesaid microorganisms until the soil is free of said nitroaromatic compounds.

Injection of microoorganisms is carried out to reach high cellular density in the soil (of the order of 10⁴ to 10⁸ bacteria per gram of soil) with the object of facilitating the removal of the pollutant. If necessary, the soil can be supplemented with a source of C and/or phosphate to facilitate the survival of the bacteria.

Finally, a third application of the bacteria provided by this invention is their employment in destroying excess TNT in arsenals or explosives production plants. For this purpose there is added to the bacteria cultures provided by this invention solid TNT or TNT adequately dissolved and neutralized (if required), following a procedure similar to that previously described above for the removal of nitroaromatic compounds contained in effluents.

The advantages that derive from the processes for removing biologically the aforementioned nitroaromatic compounds provided by this invention may be summarized as follows:
i) high specificity in the removal of 2,4-DNT, 2,6-DNT and TNT;
ii) they function over a wide range of concentrations of pollutants, individually or in blends (from 0.1 g/l to saturation and supersaturation of same pollutants), in the presence or absence of other organic or inorganic sources of N; and
iii) they display a high degree of versatility since they can be used in situ to remove said nitroaromatic compounds in the effluents of an explosives plant or in soil and water polluted with these compounds.

Specific applications of the present invention are illustrated by the following examples, which should not be considered limitative of the scope of the invention.

### EXAMPLE 1 Isolation of the bacteria

A sample of 10 g of soil from El Paramo de Masa, Burgos province, was suspended in a type-9 minimum culture medium without a nitrogen source, in a 1:10 ratio (w/v). From said suspension were taken 6 identical aliquots to which were added:

| Sample | 2,4-DNT (mg/l) | 2,6-DNT (mg/l) | TNT (mg/l) | Fructose (g/l) |
|---|---|---|---|---|
| I | 100 | --- | --- | --- |
| II | --- | 100 | --- | --- |
| III | --- | --- | 100 | --- |
| IV | 100 | --- | --- | 5 |
| V | --- | 100 | --- | 5 |
| VI | --- | --- | 100 | 5 |

The resulting suspensions I to VI were incubated at a temperature of between 25° and 30°C, with optional agitation, for one week. Subsequently, suitable dilutions of said suspensions were then applied to the plates of a selective solid medium composed of M9 medium (without N source), 1.5% (w/v) noble agar and
100 mg/ml of 2,4-DNT (Sample I), or
100 mg/ml of 2,6-DNT (Sample II), or
100 mg/ml of TNT (Sample III), or
100 mg/ml of 2,4-DNT + 5 g/l fructose (Sample IV), or
100 mg/ml of 2,6-DNT + 5 g/l fructose (Sample V), or
100 mg/ml of TNT + 5 g/l fructose (Sample VI).

After incubating for between 24 hours and one week, the isolated colonies were purified and their phenotype established by cultivating them in the above-mentioned media.

Following a microbiological type analysis, the isolated bacteria in all cases were classified as belonging to the genus Pseudomonas, and probably belonging to the species Pseudomonas aeruginosa and exhibited the characteristics mentioned previously in the description of the invention. A culture of these bacteria called Pseudomonas aeruginosa ClonA was deposited in the National Collection of Type Cultures (NCTC) under Accession No. NCTC 12690.

### EXAMPLE 2 Use of TNT as source of N

100 ml of sterile M9 minimum medium without N source was supplemented with:
250 µl of solution of A9 micronutrients;
6 µg of iron citrate;
100 µl of an MgSO₄ 1M solution;
10 mg of TNT; and
0.5 g of fructose.

The culture was started by adding a quantity of bacteria sufficient to reach an initial turbidity at 660 nm of 0.05 units and was incubated at 30°C with agitation (150 rpm in an orbital incubator). After 24-48 hours of incubation the turbidity of the culture at 660 nm was between 1 and 2 units and the concentration of TNT in the culture medium was undetectable by HPLC analysis (25 µl of supernatant liquid from the culture was passed through a Hypersil-C-18 column and was eluted with a mixture of acetonitrile:water (60:40) at a flow rate of 0.5-1 ml/min, the nitroaromatic compound being detected with ultraviolet light at λ = 254 mm).

### EXAMPLE 3 Use of TNT as source of N

The process described in Example 2 was repeated, but using TNT at supersaturation (500 mg/ml). The TNT disappeared completely in a period of 4-5 days.

### EXAMPLE 4 Use of TNT as source of N

The process described in Example 2 was repeated, but using a system of continuous addition of TNT. The fermenter contained 250 ml of medium and the turbidity of the culture was of the order of 0.3-0.5 units at 660 mm. The saturated TNT solution was added at a flow rate of 1-7 ml/h. The TNT in the medium overflow was undetectable.

### EXAMPLE 5 Use of TNT as source of N

The process described in Example 2 was repeated, but the medium was supplemented with nitric acid and sulphuric acid so that it contained 200 mg N/l and 1000 mg S/l (with the object of simulating the typical average composition of an effluent of an explosives production plant). The pH of the medium was neutralized to a pH of between 5.5 and 7 by adding NaOH. The TNT disappeared after 24-48 hours culture at 30°C.

### EXAMPLE 6 Use of TNT as source of N

The process described in Example 4 was repeated, but 2 litres of culture medium was used and incubation was carried out in a fermenter. The operating conditions were the following:
pH: 6.85-7.28
Temperature: 27.5°C-32°C
Agitation: 150-1000 rpm
Air: 0.5 volumes/minute.

The TNT disappeared after 48 hours culture.

### EXAMPLE 7 Use of 2,6-DNT as source of N

The process described in Example 2 was repeated, but using 2,6-DNT instead of TNT. The 2,6-DNT disappeared completely in a period of 4-6 days.

### EXAMPLE 8 Use of 2,4-DNT as source of N

The process described in Example 2 was repeated, but using 2,4-DNT instead of TNT. The 2,4-DNT disappeared completely in a period of 4-6 days.

### EXAMPLE 9 Treatment of an effluent

An effluent from an explosives plant exhibited the following characteristics: Composition:
TNT: 20-100 mg/ml
Nitrates: 200 mg/ml
Sulphates: 1000 mg/ml
pH: 1.5

A volume of 1 litre of said effluent was neutralized with NaOH until a pH of 7.0 was achieved. After analysing the neutralized effluent, nutrients and micronutrients of the M9 minimum medium were added, except NH₄Cl which was omitted.

Subsequently a culture of Pseudomonas aeruginosa ClonA bacteria with an initial turbidity of 0.05 units at 660 nm was inoculated. The incubation conditions were as follows:
agitation: 150 rpm in an orbital incubator
temperature: 30°C
time: 48 h

Incubation progress was monitored by measuring periodically the contents of nitroaromatic compounds present by HPLC and it was shown that after 48 hours said nitroaromatic compounds had disappeared completely.

The object of the present invention having been described, it is declared that the following claims constitute the essence of the invention.

## Claims

1. A culture of Pseudomonas aeruginosa Clon A, said microorganism being identified by accession number NCTC 12690 and being capable of utilizing 2,4-dinitrotoluene (2,4-DNT), 2,6-dinitrotoluene (2,6-DNT) and 2,4,6-trinitrotoluene (TNT) as sources of carbon (C) and/or nitrogen (N).

2. A process for the biological removal of 2,4-dinitrotoluene (2,4-DNT), 2,6-dinitrotoluene (2,6-DNT) and 2,4,6-trinitrotoluene (TNT) contained in industrial effluents or in water polluted with said compunds, comprising the step of inoculating the effluent or polluted water to be treated with a culture of Pseudomonas aeruginosa Clon A according to claim 1 and monitoring the removal of 2,4-DNT, 2,6-DNT and TNT.

3. A process for the biological removal of 2,4-dinitrotoluene (2,4-DNT), 2,6-dinitrotoluene (2,6-DNT) and 2,4,6-trinitrotoluene (TNT) contained in topsoil polluted with said compunds, comprising the step of injecting the soil to be treated with a culture of Pseudomonas aeruginosa Clon A according to claim 1 and monitoring the removal of 2,4-DNT, 2,6-DNT and TNT.

4. A process for the biological destruction of 2,4,6-trinitrotoluene (TNT) contained in arsenals or explosives production plants comprising the step of adding said TNT to a culture of Pseudomonas aeruginosa Clon A according to claim 1.

## Patentansprüche

1. Eine Kultur von Pseudomonas aeruginosa Klon A, wobei der genannte Mikroorganismus durch die Zugangszahl NCTC 12690 gekennzeichnet ist und als Kohlenstoffquellen (C) und/oder Stickstoffquellen (N) 2,4-Dinitrotoluen (2,4-DNT), 2,6-Dinitrotoluen (2,6-DNT) und 2,4,6-Trinitrotoluen (TNT) verwendet werden kann.

2. Ein Verfahren zur biologischen Entfernung von 2,4-Dinitrotoluen (2,4-DNT), 2,6-Dinitrotoluen (2,6-DNT) und 2,4,6-Trinitrotoluen (TNT), enthalten in industriellen Ausflüssen oder in mit den genannten Verbindungen verunreinigtem Wasser, welches die Stufen der Einimpfung des zu behandelnden Ausflusses oder verunreinigten Wassers mit einer Kultur von Pseudomonas aeruginosa, Klon A, gemäss Anspruch 1, sowie der Überprüfung der Entfernung des 2,4-DNT, 2,6-DNT und TNT umfasst.

3. Ein Verfahren zur biologischen Entfernung von 2,4-Dinitrotoluen (2,4-DNT), 2,6-Dinitrotoluen (2,6-DNT) und 2,4,6-Trinitrotoluen (TNT), enthalten in einer oberflächlichen, mit den genannten Verbindungen verunreinigten Bodenschicht, welches die Stufe der Einspritzung einer Kultur von Pseudomonas aeruginosa, Klon A, in den zu behandelnden Boden, gemäss Anspruch 1, sowie die Überprüfung der Entfernung des 2,4-DNT, 2,6-DNT und TNT umfasst.

4. Ein Verfahren zur biologischen Zerstörung von 2,4,6-Trinitrotoluen (TNT), enthalten in Arsenalen oder Herstellungswerken für Sprengstoffe, welches die Stufe der Zugabe des genannten TNT zu einer Kultur von Pseudomonas aeruginosa, Klon A, gemäss Anspruch 1 umfasst.

## Revendications

1. Une culture de Pseudomonas aeruginosa Clone A, ce micro-organisme étant identifié par le numéro d'accès NCTC 12690 et étant capable d'utiliser 2,4-dinitrotoluène (2,4-DNT), 2,6-dinitrotoluène (2,6-DNT) et 2,4,6-trinitrotoluène (TNT) comme source de carbone (C) et/ou d'azote (N).

2. Un procédé pour l'élimination biologique de 2,4-dinitrotoluène (2,4-DNT), 2,6-dinitrotoluène (2,6-DNT) et 2,4,6-trinitrotoluène (TNT) contenu dans des effluents industriels ou dans de l'eau polluée par ces composants, qui comprend les étapes d'inoculer dans l'effluent ou l'eau polluée à traiter une culture de Pseudomonas aeruginosa, Clone A, conformément à la revendication 1 et de contrôler l'élimination de 2,4-DNT, 2,6-DNT et TNT.

3. Un procédé pour l'élimination biologique de 2,4-dinitrotoluène (2,4-DNT), 2,6-dinitrotoluène (2,6-DNT) et 2,4,6-trinitrotoluène (TNT) contenu dans une couche superficielle de sol pollué par ces composants, qui comprend l'étape d'injecter dans le sol à traiter une culture de Pseudomonas aeruginosa, Clone A, conformément à la revendication 1 et de contrôler l'élimination de 2,4-DNT, 2,6-DNT et TNT.

4. Un procédé pour la destruction biologique de 2,4,6-trinitrotoluène (TNT) contenu dans des arsenaux ou des usines de production d'explosifs, qui comprend l'étape d'ajouter ce TNT à une culture de Pseudomonas aeruginosa, Clone A, conformément à la revendication 1.
